# EUROPEAN PATENT SPECIFICATION

(11) **EP 4 470 719 B1**
(45) Date of publication and mention of the grant of the patent: **24.09.2025**
(21) Application number: 23176018.2
(22) Date of filing: 30.05.2023
(51) Int. Cl.: B24B 31/02, B24B 31/06, B29C 45/00, B29C 43/32, B29C 43/02, B29C 71/00, H04R 25/00, A61F 11/08, B29C 59/02, B29C 37/02

(54) **METHOD TO PRODUCE POLYMERIC PRODUCTS**
VERFAHREN ZUR HERSTELLUNG POLYMERER PRODUKTE
MÉTHODE DE PRODUCTION DE PRODUITS POLYMÈRES

(43) Date of publication of application: 04.12.2024
(73) Proprietor: Loop, 2600 Berchem (BE)
(72) Inventor: O, Dimitri Philippe B., 2000 Antwerpen (BE); BODEWES, Maarten Thomas, 2000 Antwerpen (BE)
(74) Representative: Hoyng Rokh Monegier B.V.

(56) References cited:
- DE-A1- 10 162 151
- KR-A- 20070 021 647
- US-A1- 2004 161 445
- US-A1- 2015 281 861
- US-A1- 2021 315 740
- US-A1- 2022 331 930

## Description

### Technical field

The invention relates to methods for manufacturing a polymer product, more specifically an elastomer product. The invention further relates to methods for manufacturing an earplug, said earplug comprising one or more parts, said polymer products, such as elastomer products being obtained by the method according to the invention.

### Prior art

In the manufacture of polymer products in general, and elastomer products in particular, it is often difficult to provide the correct and desired surface properties and appearance for the outer sides of the product. The outer walls of the product generally take on the surface appearance of the walls of the die in which the product is made, e.g. cast or sprayed. Providing an elastomer product with a uniformly dull or matt appearance is often difficult or even impossible. DE10162151A1 discloses a method to deburr polymeric articles. The polymeric articles are rotated in a perforated drum, while being blasted by fine water droplets at high pressure. Optionally fine ceramic particles are added to the water during blasting. The water is drained of from the drum during blasting and recycled.

### Summary of the invention

The aim of the invention is to make polymer products, preferably elastomer products, with an outer side that is dull or matt, for example matted or made dull.

According to the first aspect of the invention, a method is provided for manufacturing polymer products. The method comprises the consecutive steps of:
- forming one or more intermediate polymer products, the polymer product is formed from elastomeric polymer;
- collecting said intermediate polymer products in a drum and adding abrasive objects and a liquid to the drum, said abrasive objects are ceramic objects having an average size varying between 1 mm and 30 mm, said liquid comprising water;
- tumbling the one or more intermediate polymer products with said abrasive objects in the presence of said liquid in said drum after said collecting said intermediate polymer products abrasive objects and a liquid in the drum.
- separating said intermediate polymer products from said abrasive objects and said liquid after tumbling said intermediate polymer products abrasive objects and a liquid in the drum.

The method has the advantage that the polymer product, for example elastomer, on the external surface is not only free from flash or annoying edges formed during forming of the product. In addition, the external surface of the polymer product, for example elastomer product is matted by this specific tumbling treatment. Polymer products, for example elastomer products that are manufactured, for example by injection moulding or compression moulding, often have a glossy external surface, possibly over only a portion of their external surface. This may be perceived as annoying or of low quality. The method according to the invention may overcome this partly or completely, since surprisingly, tumbling with abrasive objects in the presence of a liquid has a matting action on the external surface of the polymer product, for example elastomer product. The matting action, especially for non-elastomeric polymer products may on the other hand ensure that coating or finishing layers applied later adhere better and more uniformly to the surface, which is then beneficial for the life and quality of the finished product.

According to some embodiments, the method may comprise tumbling during a period between half an hour and two hours.

According to some embodiments the liquid may comprise water and soap.

According to some embodiments, the polymer product may be formed from silicone polymer.

The silicone polymer may be a polysiloxane polymer. As an alternative elastomeric polymer, the polymer product may be manufactured from thermoplastic elastomers (TPE), for example thermoplastic polyurethane (TPU). Further alternatives are for example polyvinyl chloride (PVC) or rubber.

The method according to the invention has the advantage that these polymer products can be matted in a particularly efficient manner over their entire outer side or external surface.

According to some embodiments, the polymer product may be formed by compression moulding.

In this forming process, soft, for example heated, polymer, for example elastomeric polymer is pressed between two die parts in a mould. The soft polymer is placed or poured in a first part of the die. The second part of the die is pressed on and in the first part and the polymer spreads in the opening and the open space between the two die parts. In this way, the product is given its final form. Possibly a small amount of polymer flows between the joint of the first and second die part. This may lead to formation of flash or an edge. This flash or edge often has to be removed. This may be done manually. The external surface of the formed product also takes on the surface condition of the inside of the die parts. These are often polished, and therefore smooth, to make it easy to remove the polymer product from the mould. This gives a partially or completely glossy external surface on the formed product. The tumbling step of the method according to the invention removes the flash and edges, but will also, surprisingly, produce a matt finish on the polymer, for example elastomer, external surface.

Ceramic objects as abrasive objects are very aggressive and are used for polishing metal surfaces. It is therefore surprising that these objects are suitable for producing matting of polymer, for example elastomer surfaces.

The ceramic objects may be of various shapes. The average size of the objects may vary between 1 mm and 30 mm. The ceramic objects may be cylindrical, cylindrical with slanting cut-off top and bottom faces, cylindrical with an elliptical radial cross-section, whether or not with slanting cut-off top and bottom faces, triangular whether or not with perpendicular or slanting vertical side walls, star-shaped whether or not with perpendicular or slanting vertical side walls, conical, wedge-shaped, pyramidal, etc. Suitable abrasive objects are for example abrasive objects from Kramer Industries Inc., USA. Preferably, abrasive objects with large dimensions are selected, for example with an average size from 15 to 30 mm, for example 18 mm to 25 mm, for example about 20 mm.

Preferably the ratio of abrasive objects relative to the liquid is in the range from 0.5:1 to 1:0.5. The weight ratio of abrasive objects and polymer products is preferably in the range from 5:1 to 1:5, such as in the range from 4:1 to 1:4, more preferably in the range from 2.5:1 to 1:3.5.

Tumbling preferably takes place in batch mode; one batch preferably comprises two to three tumbling operations which in each case last about half an hour. A batch may comprise up to 30000 or even up to 50000 products, in a tumbling drum of for example 120 litres.

According to some embodiments, the product may comprise an open torus with a cylindrical body thereon, which has an upper side connected to the torus.

A torus is a three-dimensional body of revolution, which arises on rotating a circle about a line or axis that is located in the plane of the circle. Open tori are also called doughnut shapes. In alternative embodiments, the product may comprise a torus-like body of revolution, obtained by revolving a closed 2D figure about an axis, for example an ellipse or a polygon, optionally with rounded corners, for example a triangle, quadrilateral, pentagon or hexagon. In the case of open torus-like bodies of revolution the axis does not intersect the rotated figure.

According to some embodiments, the axis of the cylindrical body and the axis of the torus may make an angle (α) between 0° and 60°.

Preferably the axis of the cylindrical body and the axis of the torus make an angle (α) between 0° and 45°. The angle between the two axes is defined as the smallest angle measurable between these two axes in perpendicular projection of the axes on a plane that is parallel to both axes. The axis of the cylindrical body is the axis of the straight part of the cylinder, and does not take into account the direction of the axis in the part in abutment with the torus. An angle of 0° gives an axis of the cylindrical body and the axis of the torus that are parallel. An angle of 90° is formed when the axis of the cylindrical body extends radially relative to the torus.

Since the centre plane of the torus is perpendicular to the axis of the torus, the angle between the centre plane of the torus and the axis of the cylindrical body is preferably between 90° and 150°, for example 100°. This angle is measured in the plane defined by the axis of the cylindrical body, said plane being perpendicular to the centre plane of the torus.

The cylindrical body does not necessarily need to be straight over its whole length, but may be partly curved. The outside diameter of the cylindrical body is preferably equal to the diameter of the circle whose rotation forms the torus.

The torus has a minimum or inside diameter preferably between 5 and 15 mm and a maximum or outside diameter preferably between 10 and 20 mm. The rotating circle of the torus preferably has a diameter from 2 to 6 mm.

According to some embodiments, the product may provide the outer-ear part of the earplug.

According to some embodiments, the product may be a body of revolution that arises by rotating a flat C shape about a line or axis that is located in the plane of the C shape; the opening of the C shape is oriented towards the outer side of the body of revolution. Preferably it is an open body of revolution, formed by rotating the C shape about a line or axis that is located in the plane of the C shape but said axis does not intersect the C shape.

The body of revolution is thus for example a circular object with a C-shaped radial cross-section; the opening of the C shape is oriented towards the outer side of the circular object. The open body of revolution has a central circular opening.

The open body of revolution has a minimum or inside diameter preferably between 3 and 6 mm and a maximum or outside diameter on a first side of the body of revolution preferably between 10 and 13 mm. On the second side of the body of revolution, the maximum or outside diameter may be equal to that of the one first side of the body of revolution, but is preferably smaller, for example between 9 and 11 mm. The thickness of the body of revolution, i.e. the distance between the two sides of the body of revolution, also called upper side and underside, is preferably between 3 and 5 mm. The wall thickness of the body of revolution is preferably between 2 and 5 mm.

According to some embodiments, the polymer product may be the plug of an earplug.

The plug is also called the earpiece of the earplug. The plug may be of conical configuration, optionally with a central cavity in which a tubular or cylindrical body may be inserted or plugged-in.

According to some embodiments, the product may form part of an earplug.

According to some embodiments, a dirt-repellent coating may be applied after tumbling.

This coating has the additional advantage that the surface remains clean, even if the product is used as part of an earplug.

Preferably, the dirt-repellent coating is a silicone coating, for example a silicone coating based on methylated siloxanes and pyrogenic silicic acid.

According to some embodiments, the coating may be applied for example by spraying the coating or by some other suitable manner of applying a coating, such as vacuum coating, spraying, dipping, and similar techniques.

According to a second aspect of the invention, a method is provided for manufacturing an earplug, said method comprising the following steps:
a) forming an outer-ear part by any one of the methods according to the first aspect of the invention;
b) forming a plug;
c) fitting the plug on the outer-ear part;
or
a) forming an outer-ear part;
b) forming a plug by any one of the methods according to the first aspect of the invention;
c) fitting a plug on the outer-ear part;
or
a) forming an outer-ear part by any one of the methods according to the first aspect of the invention;
b) forming a plug by any one of the methods according to the first aspect of the invention;
c) fitting the plug on the outer-ear part.

The product obtained by the method of the first aspect of the invention may be a body of revolution with a C-shaped radial cross-section, the opening of the C shape being oriented towards the outer side of the circular object. This product may be used for the further damping or preventing of noise in an earplug, said earplug comprising a hollow open torus with a tubular body thereon, the external wall of which abuts on the torus, and wherein the hollow open torus has an opening on the inside of the hollow open torus at the level of the place where the tubular body abuts on the hollow open torus. This torus and this tubular body are preferably made of thermoplastic polymer, for example acrylonitrile butadiene styrene (ABS). The polymer product, for example elastomer product, obtained by a method of the invention can be plugged into the open shape of the hollow open torus. As a result, sound will be damped or blocked in the hollow open torus, and thus in the earplug. The curvature on the inside of the C shape is preferably the same as the curvature on the inside of the hollow open torus, and the smallest diameter of the C shape is preferably equal to the smallest diameter on the inside of the hollow open torus.

The independent claims and the dependent claims describe specific and preferred characteristic features of the embodiments of the invention. Features of the dependent claims may be combined with features of the independent claims and of the dependent claims, or with features described above and/or hereunder, in any suitable manner such as would be clear to a person skilled in the art.

The aforementioned and other characteristic features, properties and advantages of the present invention will be explained with the aid of the following examples of embodiments, optionally in conjunction with the drawings.

The description of these examples of embodiments is given for purposes of explanation, without the intention of limiting the scope of the invention, which is defined by the claims. The reference numbers in the description given hereunder refer to the drawings. The same reference numbers in possibly different figures refer to identical or similar elements.

### Brief description of the figures

For better illustration of the features of the invention, some preferred embodiments are described hereunder, as examples without any limiting character, referring to the appended drawings:
- Fig. 1 is a schematic representation of an embodiment of a method according to the invention.
- Figs. 2 and 3 show a schematic representation of a first product formed by the method of Fig. 1. Fig. 2 is a side view, whereas Fig. 3 is a top view of the product.
- Figs. 4 and 5 show a schematic representation of a first product formed by the method of Fig. 1. Fig. 4 is a top view, whereas Fig. 5 is a side view of the product.

The same reference numbers refer to similar or identical features or objects in the various figures.

### Description of examples of embodiments

The present invention is described hereunder making use of specific embodiments.

It should be noted that the term "comprising", for example as used in the claims, is not to be interpreted in a limiting sense, limiting to the elements, features and/or steps stated thereafter. The term "comprising" does not exclude the presence of other elements, features or steps.

Thus, the scope of an expression "an object comprising the elements A and B" is not limited to an object that only contains the elements A and B. The scope of an expression "a method comprising the steps A and B" is not limited to a method that only contains the steps A and B.

In the sense of the present invention, these expressions only signify that the relevant elements or steps for the invention are the elements or steps A and B.

In the specification given hereunder, reference is made to "an embodiment" or "the embodiment". This reference signifies that a specific element or feature, described on the basis of this embodiment, is comprised in at least this one embodiment.

The occurrence of the terms "in an embodiment" or "in the embodiment" at various places in this description does not, however, necessarily refer to the same embodiment, although it may well, however, refer to one and the same embodiment.

Moreover, the properties or the features may be combined in any suitable manner in one or more embodiments, as would be clear to a person skilled in the art.

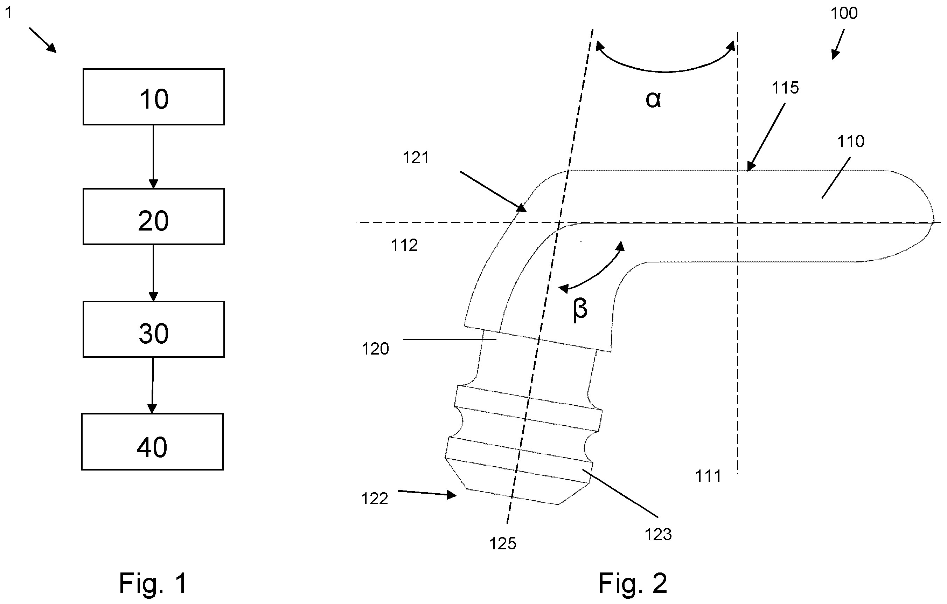

An example of a method 1 for manufacturing polymer products according to the invention is shown schematically in Fig. 1. In step 10, a silicone intermediate product is formed by compression moulding.

In step 20, a number of such products are collected together in a drum of about 120 litres capacity. Abrasive objects, for example ceramic objects with an average size of about 20 mm are added, as well as water. The ratio of abrasive objects relative to water is preferably about 1:1. About 20 litres of abrasive objects are added for 12 to 20 kg of intermediate products. The mixture is tumbled until the silicone intermediate products have a matt surface. The time required may vary, but is generally between half an hour and two hours.

After tumbling, in step 30 the intermediate products are separated from the water and the abrasive objects, and spread out on a grating. Then, in step 40, the intermediate products are sprayed with a dirt-repellent fluid that is based on methylated siloxanes and pyrogenic silicic acid, together with an organic vehicle. The vehicle evaporates and a dirt-repellent coating is thus formed on the manufactured products.

The result is a product made of polymer, for example elastomeric polymer, more specifically a silicone product, which has a uniformly matt appearance on the external surfaces.

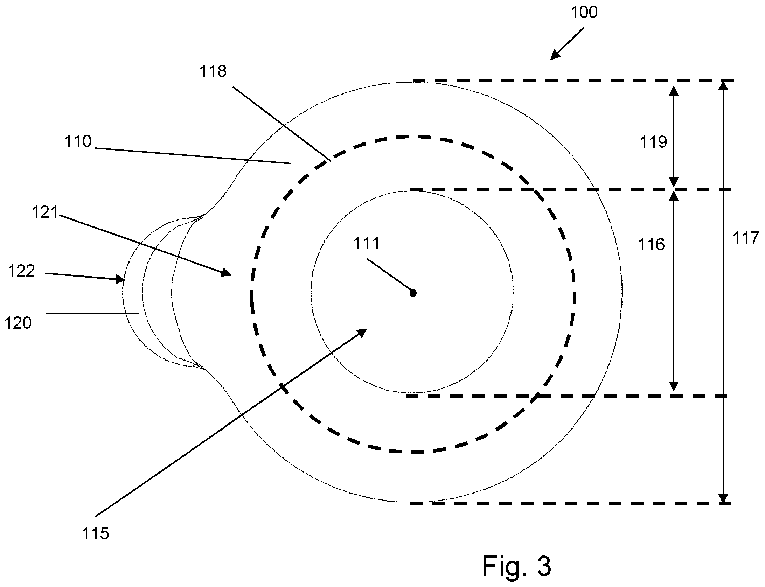

A first product 100 that forms part of an earplug, said part being manufactured by method 1 described above, is shown in Fig. 2 and Fig. 3.

The product 100 comprises an open torus 110 with a cylindrical body 120 thereon, which has an upper side 121 that abuts on the torus. The opposite side 122 comprises coupling ribs 123 for coupling to another product. The open torus is formed by rotating a circle with diameter 117 about a central axis 111, wherein the centre of the circle itself describes a circle 118 about the axis 111.

A cylindrical body 120 is fastened to the torus. The angle α between the axis 125 of the cylindrical body and the axis 111 of the torus is about 10°. The angle β between the centre plane 112 of the torus 110 and the axis 125 of the cylindrical body 120 is about 100°.

The cylindrical body is slightly curved near the point where the cylindrical body abuts on the torus 110. At the level of the abutment of the cylindrical body and the torus 110, the outside diameter of the cylindrical body is equal to the diameter of the circle whose rotation forms the torus.

The torus 110 has a central opening 115 with a minimum or inside diameter 116 between 5 and 15 mm; the torus has a maximum or outside diameter 117 between 10 and 20 mm.

This product 100 is the outer-ear part of an earplug. The plug of the earplug, which is conical and which has a central cavity to receive the cylindrical body, is pushed onto the side 122 of the cylindrical body. The inside of the plug has recesses that correspond to the coupling ribs 123. The plug may also be made from the same material and by the same method 1. The earplug thus formed is an earplug that blocks almost all sound.

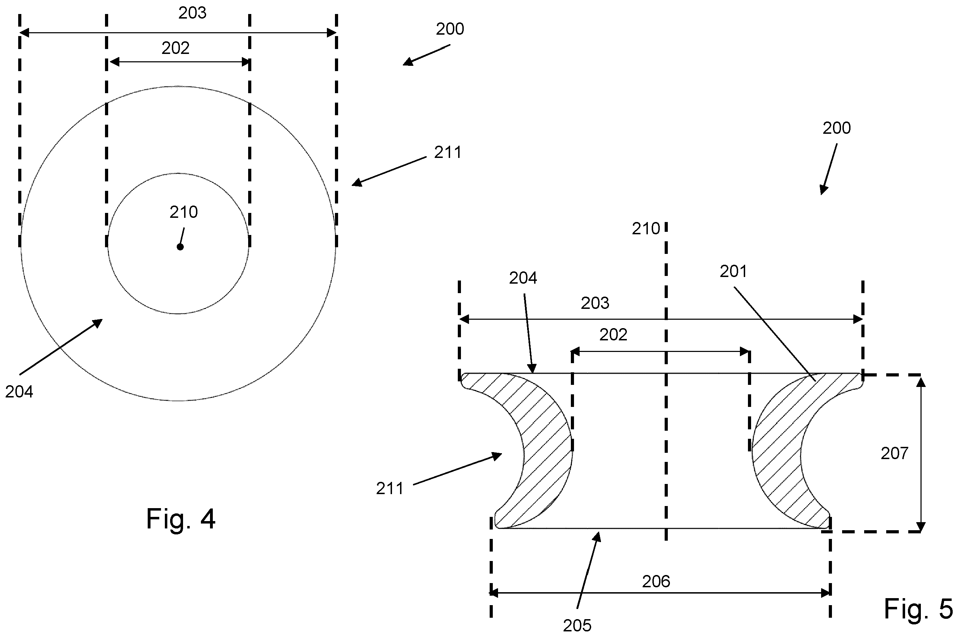

A second product 200 that forms part of an earplug, said part being manufactured by method 1 described above, is shown in Fig. 4 and Fig. 5.

The product 200 is a body of revolution, which arises through rotating a flat C shape 201 in a circle about an axis 210 that is located in the plane of the C shape and wherein the opening of the C shape is oriented towards the outer side 211 of the body of revolution.

The product 200 is thus a circular object with a C-shaped radial cross-section. The opening of the C shape is oriented towards the outer side of the circular object.

The body of revolution has a minimum or inside diameter 202 between 3 and 6 mm and a maximum or outside diameter 203 on a first side 204 of the body of revolution between 10 and 13 mm. On the second side 205 of the body of revolution, the maximum or outside diameter 206 is for example between 9 and 11 mm. The thickness 207 of the body of revolution is between 3 and 5 mm.

This product 200 may form part of an earplug. A product with the same shape as shown for product 100 may be manufactured from rigid polymer and may be used as the outer-ear part of an earplug. The torus and the cylindrical body may be hollow on the inside, and on the inside of the torus a perforation may be provided at the level of the abutment with the cylindrical body. This earplug functions as a sound damper, rather than as a block to all sound. Product 200 can be plugged into the opening of the open torus, so as to block all sound.

It is clear that although the embodiments and/or the materials for supplying embodiments according to the present invention are discussed, various alterations or modifications may be made without departing from the scope of the present invention, as defined by the claims. The present invention is not in any way limited to the embodiments described above, but may be carried out according to many variants while remaining within the scope of the present invention, defined by the claims.

## Claims

1. Method for manufacturing polymer products, said method comprising the consecutive steps of:
• forming one or more intermediate polymer products, the polymer product is formed from elastomeric polymer;
• collecting said intermediate polymer products in a drum and adding abrasive objects and a liquid to the drum, said abrasive objects are ceramic objects having an average size varying between 1 mm and 30 mm, said liquid comprising water;
• tumbling the one or more intermediate polymer products with said abrasive objects in the presence of said liquid in said drum after said collecting said intermediate polymer products abrasive objects and a liquid in the drum.
• separating said intermediate polymer products from said abrasive objects and said liquid after tumbling said intermediate polymer products abrasive objects and a liquid in the drum.

2. Method according to claim 1, wherein the method comprises tumbling during a period between half an hour and two hours.

3. Method according to any one of the preceding claims, wherein the liquid comprises water and soap.

4. Method according to one of the preceding claims, wherein the ratio of abrasive objects relative to the liquid is in the range from 0.5:1 to 1:0.5.

5. Method according to one of the preceding claims, wherein the weight ratio of abrasive objects and polymer products is in the range from 5:1 to 1:5.

6. Method according to any one of the preceding claims, wherein the polymer product is formed from silicone polymer.

7. Method according to any one of the preceding claims, wherein the polymer product is formed by compression moulding.

8. Method according to any one of the preceding claims, wherein the product comprises an open torus with a cylindrical body thereon, which has an upper side that abuts on the ring.

9. Method according to claim 8, wherein the axis of the cylindrical body and the axis of the torus make an angle (α) between 0° and 60°.

10. Method according to claim 8 or 9, wherein the product provides the outer-ear part of the earplug.

11. Method according to any one of claims 1 to 7, wherein the product is a body of revolution, which arises by rotating a flat C shape about a line that is located in the plane of the C shape, the opening of the C shape being oriented towards the outer side of the body of revolution.

12. Method according to any one of claims 1 to 7, wherein the polymer product is the plug of an earplug.

13. Method according to any one of the preceding claims, wherein the product forms part of an earplug.

14. Method according to any one of the preceding claims, wherein a dirt-repellent coating is applied after tumbling.

15. Method according to claim 14, wherein the coating is applied by spraying the coating.

16. A method for manufacturing an earplug, said method comprising the following steps:
a) forming an outer-ear part by a method according to any one of claims 1 to 15;
b) forming a plug;
c) fitting the plug on the outer-ear part;
or
a) forming an outer-ear part;
b) forming a plug by a method according to any one of claims 1 to 15;
c) fitting a plug on the outer-ear part;
or
a) forming an outer-ear part by a method according to any one of claims 1 to 15;
b) forming a plug by a method according to any one of claims 1 to 15;
c) fitting the plug on the outer-ear part.

## Patentansprüche

1. Verfahren zum Herstellen von Polymerprodukten, das Verfahren umfassend die aufeinanderfolgenden Schritte:
• Ausbilden eines oder mehrerer Polymerzwischenprodukte, wobei das Polymerprodukt aus einem elastomeren Polymer ausgebildet ist;
• Sammeln der Polymerzwischenprodukte in einer Trommel und Hinzufügen von Schleifobjekten und einer Flüssigkeit zu der Trommel, wobei die Schleifobjekte Keramikobjekte sind, die eine durchschnittlichen Größe aufweisen, die zwischen 1 mm und 30 mm variiert, die Flüssigkeit umfassend Wasser;
• Wirbeln des einen oder der mehreren Polymerzwischenprodukte mit den Schleifobjekten in Gegenwart der Flüssigkeit in der Trommel nach dem Sammeln der Polymerzwischenprodukte, der Schleifobjekte und einer Flüssigkeit in der Trommel.
• Trennen der Polymerzwischenprodukte von den Schleifobjekten und der Flüssigkeit nach dem Wirbeln der Polymerzwischenprodukte, der Schleifobjekte und einer Flüssigkeit in der Trommel.

2. Verfahren nach Anspruch 1, wobei das Verfahren das Wirbeln während einer Zeitspanne zwischen einer halben Stunde und zwei Stunden umfasst.

3. Verfahren nach einem der vorstehenden Ansprüche, wobei die Flüssigkeit Wasser und Seife umfasst.

4. Verfahren nach einem der vorstehenden Ansprüche, wobei das Verhältnis von den Schleifobjekten zu der Flüssigkeit im Bereich von 0,5 : 1 bis 1 : 0,5 liegt.

5. Verfahren nach einem der vorstehenden Ansprüche, wobei das Gewichtsverhältnis von den Schleifobjekten und den Polymerprodukten im Bereich von 5 : 1 bis 1 : 5 liegt.

6. Verfahren nach einem der vorstehenden Ansprüche, wobei das Polymerprodukt aus Silikonpolymer ausgebildet ist.

7. Verfahren nach einem der vorstehenden Ansprüche, wobei das Polymerprodukt durch Formpressen ausgebildet ist.

8. Verfahren nach einem der vorstehenden Ansprüche, wobei das Produkt einen offenen Torus mit einem zylindrischen Körper darauf umfasst, der eine Oberseite aufweist, die an den Ring angrenzt.

9. Verfahren nach Anspruch 8, wobei die Achse des zylindrischen Körpers und die Achse des Torus einen Winkel (α) zwischen 0° und 60° bilden.

10. Verfahren nach Anspruch 8 oder 9, wobei das Produkt den Außenohrteil des Ohrstöpsels bereitstellt.

11. Verfahren nach einem der Ansprüche 1 bis 7, wobei das Produkt ein Rotationskörper ist, der durch Drehen einer flachen C-Form um eine Linie entsteht, die sich in der Ebene der C-Form befindet, wobei die Öffnung der C-Form in Richtung der Außenseite des Rotationskörpers ausgerichtet ist.

12. Verfahren nach einem der Ansprüche 1 bis 7, wobei das Polymerprodukt der Stöpsel eines Ohrstöpsels ist.

13. Verfahren nach einem der vorstehenden Ansprüche, wobei das Produkt Teil eines Ohrstöpsels ausbildet.

14. Verfahren nach einem der vorstehenden Ansprüche, wobei nach dem Wirbeln eine schmutzabweisende Beschichtung aufgebracht wird.

15. Verfahren nach Anspruch 14, wobei die Beschichtung durch Sprühen der Beschichtung aufgebracht wird.

16. Verfahren zum Herstellen eines Ohrstöpsels, das Verfahren umfassend die folgenden Schritte:
a) Ausbilden eines Außenohrteils durch ein Verfahren nach einem der Ansprüche 1 bis 15;
b) Ausbilden eines Stöpsels;
c) Anbringen des Stöpsels an dem Außenohrteil;
oder
a) Ausbilden eines Außenohrteils;
b) Ausbilden eines Stöpsels durch ein Verfahren nach einem der Ansprüche 1 bis 15;
c) Anbringen eines Stöpsels an dem Außenohrteil;
oder
a) Ausbilden eines Außenohrteils durch ein Verfahren nach einem der Ansprüche 1 bis 15;
b) Ausbilden eines Stöpsels durch ein Verfahren nach einem der Ansprüche 1 bis 15;
c) Anbringen des Stöpsels an dem Außenohrteil.

## Revendications

1. Procédé permettant de fabriquer des produits polymères, ledit procédé comprenant les étapes consécutives consistant à :
• former un ou plusieurs produits polymères intermédiaires, le produit polymère étant formé de polymère élastomère ;
• recueillir lesdits produits polymères intermédiaires dans un tambour et ajouter des objets abrasifs et un liquide au tambour, lesdits objets abrasifs étant des objets céramiques ayant une taille moyenne variant entre 1 mm et 30 mm, ledit liquide comprenant de l'eau ;
• réaliser le tonnelage du ou des produits polymères intermédiaires avec lesdits objets abrasifs en présence dudit liquide dans ledit tambour après avoir recueilli lesdits produits polymères intermédiaires, les objets abrasifs et un liquide dans le tambour ;
• séparer lesdits produits polymères intermédiaires desdits objets abrasifs et dudit liquide après avoir réalisé le tonnelage desdits produits polymères intermédiaires, des objets abrasifs et d'un liquide dans le tambour.

2. Procédé selon la revendication 1, dans lequel le procédé comprend le tonnelage pendant une période comprise entre une demi-heure et deux heures.

3. Procédé selon l'une quelconque des revendications précédentes, dans lequel le liquide comprend de l'eau et du savon.

4. Procédé selon l'une des revendications précédentes, dans lequel le rapport entre des objets abrasifs et le liquide se situe dans la plage comprise entre 0,5:1 et 1:0,5.

5. Procédé selon l'une des revendications précédentes, dans lequel le rapport pondéral entre des objets abrasifs et des produits polymères se situe dans la plage comprise entre 5:1 et 1:5.

6. Procédé selon l'une quelconque des revendications précédentes, dans lequel le produit polymère est formé à partir de polymère silicone.

7. Procédé selon l'une quelconque des revendications précédentes, dans lequel le produit polymère est formé par moulage par compression.

8. Procédé selon l'une quelconque des revendications précédentes, dans lequel le produit comprend un tore ouvert sur lequel repose un corps cylindrique dont une face supérieure vient en butée sur l'anneau.

9. Procédé selon la revendication 8, dans lequel l'axe du corps cylindrique et l'axe du tore forment un angle (α) compris entre 0° et 60°.

10. Procédé selon la revendication 8 ou 9, dans lequel le produit fournit la partie oreille externe du bouchon d'oreille.

11. Procédé selon l'une quelconque des revendications 1 à 7, dans lequel le produit est un corps de révolution, qui résulte de la rotation d'une forme plate en C autour d'une ligne qui est située dans le plan de la forme en C, l'ouverture de la forme en C étant orientée vers le côté externe du corps de révolution.

12. Procédé selon l'une quelconque des revendications 1 à 7, dans lequel le produit polymère est le bouchon d'un bouchon d'oreille.

13. Procédé selon l'une quelconque des revendications précédentes, dans lequel le produit fait partie d'un bouchon d'oreille.

14. Procédé selon l'une quelconque des revendications précédentes, dans lequel un revêtement anti-salissures est appliqué après le tonnelage.

15. Procédé selon la revendication 14, dans lequel le revêtement est appliqué par pulvérisation du revêtement.

16. Procédé permettant de fabriquer un bouchon d'oreille, ledit procédé comprenant les étapes suivantes consistant à :
a) former une partie oreille externe par un procédé selon l'une quelconque des revendications 1 à 15 ;
b) former un bouchon ;
c) mettre en place le bouchon sur la partie oreille externe ;
ou
a) former une partie oreille externe ;
b) former un bouchon par un procédé selon l'une quelconque des revendications 1 à 15 ;
c) mettre en place un bouchon sur la partie oreille externe ;
ou
a) former une partie oreille externe par un procédé selon l'une quelconque des revendications 1 à 15 ;
b) former un bouchon par un procédé selon l'une quelconque des revendications 1 à 15 ;
c) mettre en place le bouchon sur la partie oreille externe.
